# EUROPEAN PATENT APPLICATION

(11) **EP 3 875 076 A1**
(43) Date of publication of application: **08.09.2021**
(21) Application number: 21159550.9
(22) Date of filing: 26.02.2021
(51) Int. Cl.: A61K 9/16, A61K 31/00, A61K 36/00

(54) **WATER-SOLUBLE MICROENCAPSULATED CANNABINOID EXTRACT POWDER AND METHOD OF MAKING THE SAME**

(30) Priority: 27.02.2020 US 202062982512 P; 23.02.2021 US 202117182871
(71) Applicant: Prinova Flavors LLC, Carol Stream, IL 60188 (US)
(72) Inventor: Paredes, Stephanie, IL 60188 (US); Amann, Alexander, IL 60188 (US)
(74) Representative: Somervell, Thomas Richard

(57) **Abstract**

A water-soluble microencapsulated cannabinoid powder consisting essentially of a cannabinoid composition and at least one gum Acacia, and a method of making the same are provided.

## Description

### BACKGROUND

The present disclosure relates to cannabinoid extract composition, and more particularly to water-soluble encapsulated cannabidiol (CBD) powder.

CBD may be used as an ingredient for food, beauty products, supplements and other similar applications. CBD is oil soluble. The oil soluble nature of CBD presents challenges for using CBD in water-soluble applications. CBD encapsulations in fats are known. However, fat encapsulated CBD powders are typically not water soluble or have a relatively low water solubility, and thus, less bioavailable when consumed.

The present disclosure provides a water-soluble microencapsulated CBD powder that contains a relatively high concentration of CBD and has a relatively high water solubility, and a method of making the same according to various embodiments.

### BRIEF SUMMARY

In one aspect, a method of making a water-soluble microencapsulated cannabinoid powder is provided. The method may include the steps of solubilizing a cannabinoid composition in a hydro-alcoholic solution to form a cannabinoid emulsion, stabilizing the cannabinoid emulsion by adding a hydrocolloid, mixing the cannabinoid emulsion and the hydrocolloid to form a cannabinoid-hydrocolloid emulsion, drying the cannabinoid-hydrocolloid emulsion, and forming a water-soluble microencapsulated cannabinoid powder.

In an embodiment, the hydro-alcoholic solution may comprise about 50% ethanol and about 50% water. For example, the hydro-alcoholic solution may be prepared using about 50% 190 proof cane ethanol and about 50% water.

The method may further comprise the step of hydrating the hydrocolloid in water to forma a hydrocolloid pre-emulsion. In such an embodiment, the step of mixing the cannabinoid emulsion and the hydrocolloid may include mixing and homogenizing the cannabinoid emulsion and the hydrocolloid pre-emulsion under a high pressure. For example, the mixture of the cannabinoid emulsion and the hydrocolloid pre-emulsion may be homogenized under a pressure of about 2,000 psi to about 5,000 psi.

In some embodiments, the hydrocolloid may comprise at least two varietals of gum Acacia. For example, the hydrocolloid may comprise gum Acacia Seyal and gum Acacia Senegal.

The step of drying the cannabinoid-hydrocolloid emulsion may include processing the cannabinoid-hydrocolloid emulsion through a spray dryer. In an embodiment, an inlet temperature of the spray dryer may be set at about 170°C to about 175°C and an outlet temperature of the spray dryer may be set at about 70°C to about 75°C.

In some embodiments, the step of forming a water-soluble microencapsulated cannabinoid powder may comprise sieving the dried cannabinoid-hydrocolloid emulsion using a mesh, for example, a 18 US mesh.

The water-soluble microencapsulated cannabinoid powder made according to any of foregoing embodiments may consist essentially of the cannabinoid composition and the hydrocolloid, wherein the cannabinoid composition is included in a concentration of about 10% w/w to about 20% w/w.

In another aspect, a water-soluble microencapsulated cannabinoid powder consisting essentially of a cannabinoid composition and at least one gum Acacia is provided. In an embodiment, the at least one gum Acacia may include gum Acacia Seyal and gum Acacia Senegal. The water-soluble microencapsulated cannabinoid powder may contain the cannabinoid composition in a concentration of about 10% w/w to about 20% w/w.

Other aspects, objectives and advantages will become more apparent from the following detailed description.

### DETAILED DESCRIPTION

While the present disclosure is susceptible of embodiment in various forms, there will hereinafter be described presently preferred embodiments with the understanding that the present disclosure is to be considered an exemplification and is not intended to limit the disclosure to the specific embodiments illustrated.

A process and composition for producing a water-soluble microencapsulated cannabinoid extract powder are disclosed. The water-soluble microencapsulated cannabinoid extract powder is also referred to herein as water-dispersible cannabinoid powder, water-soluble cannabidiol (CBD) powder, water-dispersible microencapsulated CBD powder, and other similar terms. The process or method for manufacturing a flowable and water-dispersible CBD powder may include solubilizing a cannabinoid composition in a hydro-alcoholic solution to form a mixture. In an embodiment, a hydro-alcoholic solution may comprise about 50% alcohol, such as ethanol, and 50% water.

The mixture of the cannabinoid composition and hydro-alcoholic solution may be treated to form a nano-emulsion dispersed in an aqueous solution. The aqueous solution may include at least two functional excipients derived from the same plant genus. In an embodiment, functional excipients derived from the same plant genus may be two varietals of gum Acacia in a specific ratio. The combination of solubilized cannabinoid composition and hydrated gum mixture may be homogenized under high pressure and/or high shear to produce a stable nano-emulsion.

The nano-emulsion may be spray dried at about 170°C inlet and about 70°C outlet to evaporate the aqueous portion and the alcohol to form a dry powder comprising the cannabinoid composition in a bioavailable microencapsulation. The dry powder cannabinoid composition may be relatively clean label. The concentration of deliverable cannabinoid composition may be about 10% w/w to about 20% w/w, and preferably above 20% w/w, wherein the ratios of ingredients can be varied to incorporate varying loads of cannabinoid compositions.

In some embodiments, a method of making a water-dispersible cannabinoid powder may comprise the step of solubilizing a cannabinoid composition. The cannabinoid composition is typically provided as an amorphous solid, which may require solubilization to become functional. In an embodiment, the cannabinoid composition may be solubilized in a hydro-alcoholic solution comprising about 50% cane alcohol, such as 190 proof cane ethanol, and about 50% water to form an aqueous nano-emulsion without using other additives or ingredients.

The method may also comprise the step of stabilizing the aqueous nano-emulsion by adding a hydrocolloid. Suitable hydrocolloids may include, but are not limited to, varietals of gum Acacia, Agar, Alginate, Arabinoxylan, Carrageenan, Carboxymethylcellulose, Cellulose, Curdlan, Gelatin, Gellan β-Glucan, Guar gum, gum Arabic, Locust bean gum, Pectin, Starch, and Xanthan gum. In an embodiment, two varietals of gum Acacia, for example, gum Acacia Seyal and gum Acacia Senegal, may be used in a specific ratio and blended with the aqueous nano-emulsion to achieve optimal emulsion stability and encapsulation efficiency. For example, gum Acacia Seyal and gum Acacia Senegal may be used in a ratio of about 3:1 to about 5:1. In an embodiment, a ratio of gum Acacia Seyal to gum Acacia Senegal in the hydrocolloid is about 4:1. Tests have shown that gum Acacia Seyal can improve the encapsulation efficiency and gum Acacia Senegal can improve the emulsification stability. The hydrocolloid may be hydrated by mixing the hydrocolloid, such as the varietals of gum Acacia, in hot water under high shear to form a hydrocolloid pre-emulsion. The aqueous nano-emulsion containing the cannabinoid composition and the hydrocolloid pre-emulsion may be mixed together and homogenized under high pressure to provide a stable nano-emulsion.

Further, the method may include the step of drying the nano-emulsion comprising the cannabinoid composition and the hydrocolloid to form a water-dispersible microencapsulated cannabinoid powder. In an embodiment, the nano-emulsion comprising the cannabinoid composition and the hydrocolloid may be dried using a spray dryer. An inlet temperature of the spray dryer may be set at about 160°C to about 180°C, preferably at about 170°C to about 175°C, and outlet temperature of the spray dryer may be set at about 60°C to about 80°C, preferably at about 70°C to about 75°C.

The water-dispersible microencapsulated cannabinoid powder formed according to the various embodiments of the present disclosure may comprise about 5% w/w, about 10% w/w, about 15% w/w, about 20% w/w, about 25% w/w, about 30% w/w, about 35% w/w, or about 40% w/w of the cannabinoid composition. In an embodiment, the water-dispersible microencapsulated cannabinoid powder is a free flowing powder containing about 10% w/w to about 20% w/w of the cannabinoid composition and about 80% w/w to about 90% w/w of the gum Acacia varietals.

The water-dispersible microencapsulated cannabinoid powder may be used as an ingredient in food, cosmetic and other applications. For example, the water-dispersible microencapsulated cannabinoid powder may be used for beverage, snacks, baked goods, alcohol, powdered RTM beverage, supplements, confections, direct consumption, lotions, makeup, and any other industry where a water-dispersible cannabinoid composition may be used. The increase in water solubility is analogous to increased bioavailability of the cannabinoid composition.

The method of making the water-soluble cannabinoid powder according to various embodiments of the present discloser does not use foreign oils, emulsifiers, carriers, bulking agents, or other additives to achieve sufficient functionality. Prior cannabinoid encapsulations in fats result in a powder that is less water soluble and therefore less bioavailable when consumed.

### Example

In an embodiment, a water-dispersible microencapsulated cannabinoid powder is prepared according to the following steps:
- Heat water to approximately 25°C in a mixing tank, add gum Acacia Senegal powder and gum Acacia Seyal powder and use a high shear mixer for 30-45 minutes to hydrate the powders;
- In another tank, warm CBD distillate in a 50°C water batch, once cooled add ethanol at 50% of weight of CBD and add water at 50% of weight of CBD to form a white cloudy liquid mixture;
- Mix all of the components with high shear mixer, adjust Brix level to 20-25 degrees;
- Run the mixture through homogenizer GEA, 1st pass - 200 bar = 2900 psi, 2nd pass - 300 bar = 4300 psi;
- While continue to mix tank, begin feeding a spray dryer using parameters of 170-175°C inlet and 70-75°C outlet; and
- Pass the dried material through 18 US mesh having a nominal sieve opening of about 1,000 microns to provide the free-flowing water-dispersible microencapsulated cannabinoid powder.

The water-dispersible microencapsulated cannabinoid powder made according to this embodiment is a gum Acacia encapsulated CBD power containing CBD in a concentration of about 10% w/w to about 20% w/w, wherein the powder consisting essentially of CBD and gum Acacia is substantially free of other additives, such as oils, emulsifiers, carriers, excipients, bulking agents, etc.

All patents referred to herein, are hereby incorporated herein in their entirety, by reference, whether or not specifically indicated as such within the text of this disclosure.

In the present disclosure, the words "a" or "an" are to be taken to include both the singular and the plural. Conversely, any reference to plural items shall, where appropriate, include the singular.

From the foregoing it will be observed that numerous modifications and variations can be effectuated without departing from the true spirit and scope of the novel concepts of the present disclosure. It is to be understood that no limitation with respect to the specific embodiments illustrated is intended or should be inferred. The disclosure is intended to cover by the appended claims all such modifications as fall within the scope of the claims

## Claims

1. A method of making a water-soluble microencapsulated cannabinoid powder comprising, the steps of:
solubilizing a cannabinoid composition in a hydro-alcoholic solution to form a cannabinoid emulsion;
stabilizing the cannabinoid emulsion by adding a hydrocolloid;
mixing the cannabinoid emulsion and the hydrocolloid to form a cannabinoid-hydrocolloid emulsion;
drying the cannabinoid-hydrocolloid emulsion; and
forming a water-soluble microencapsulated cannabinoid powder.

2. The method of claim 1, wherein the hydro-alcoholic solution comprises about 50% ethanol and about 50% water.

3. The method of any of claims 1-2, wherein the hydro-alcoholic solution comprises about 50% 190 proof cane ethanol and about 50% water.

4. The method of any of claims 1-3, further comprising the step of hydrating the hydrocolloid in water to forma a hydrocolloid pre-emulsion.

5. The method of claim 4, wherein the step of mixing the cannabinoid emulsion and the hydrocolloid comprises mixing the cannabinoid emulsion and the hydrocolloid pre-emulsion, and homogenizing the cannabinoid emulsion and the hydrocolloid pre-emulsion under a pressure of about 2,000 psi to about 5,000 psi.

6. The method of any of claims 1-4, wherein the hydrocolloid comprises at least two varietals of gum Acacia.

7. The method of claim 6, wherein the hydrocolloid comprises a gum Acacia Seyal and a gum Acacia Senegal.

8. The method of any of claims 1-7, wherein the step of drying the cannabinoid-hydrocolloid emulsion comprises processing the cannabinoid-hydrocolloid emulsion through a spray dryer, wherein an inlet temperature of the spray dryer is set at about 170°C to about 175°C and an outlet temperature of the spray dryer is set at about 70°C to about 75°C.

9. The method of any of claims 1-9, wherein the step of forming a water-soluble microencapsulated cannabinoid powder comprises passing the dried cannabinoid-hydrocolloid emulsion through a mesh.

10. The method of claim 9, wherein the mesh is a 18 US mesh.

11. The method of any of claims 1-10, wherein the water-soluble microencapsulated cannabinoid powder consists essentially of the cannabinoid composition and the hydrocolloid, wherein the water-soluble microencapsulated cannabinoid powder contains the cannabinoid composition in a concentration of about 10% w/w to about 20% w/w.

12. A water-soluble microencapsulated cannabinoid powder consisting essentially of a cannabinoid composition and at least one gum Acacia.

13. The water-soluble microencapsulated cannabinoid powder of claim 12, wherein the at least one gum Acacia includes a gum Acacia Seyal and a gum Acacia Senegal.

14. The water-soluble microencapsulated cannabinoid powder of any of claims 12-13, wherein the water-soluble microencapsulated cannabinoid powder contains the cannabinoid composition in a concentration of about 10% w/w to about 20% w/w.
